(19) Europäisches Patentamt / European Patent Office / Office européen des brevets

(11) **EP 2 529 729 A1**

(12) **EUROPEAN PATENT APPLICATION**

| | |
|---|---|
| (43) Date of publication:<br>**05.12.2012 Bulletin 2012/49** | (51) Int Cl.:<br>*A61K 9/14* (2006.01)   *A61K 9/16* (2006.01)<br>*A61K 9/48* (2006.01)   *A61K 31/609* (2006.01) |
| (21) Application number: **12166566.5** | |
| (22) Date of filing: **24.08.2006** | |

| | |
|---|---|
| (84) Designated Contracting States:<br>**AT BE BG CH CY CZ DE DK EE ES FI FR GB GR HU IE IS IT LI LT LU LV MC NL PL PT RO SE SI SK TR**<br>Designated Extension States:<br>**AL BA HR MK RS** | • **Swanson, Brock**<br>**Nashville, NC 27856 (US)**<br>• **Johnson, Lorin**<br>**Palo Alto, CA 94301 (US)** |
| (30) Priority: **24.08.2005  US 711300 P** | (74) Representative: **Adam, Holger**<br>**Kraus & Weisert**<br>**Patent- und Rechtsanwälte**<br>**Thomas-Wimmer-Ring 15**<br>**80539 München (DE)** |
| (62) Document number(s) of the earlier application(s) in accordance with Art. 76 EPC:<br>**06802343.1 / 1 931 354** | |
| (71) Applicant: **Salix Pharmaceuticals, Inc.**<br>**Morrisville, NC 27560 (US)** | Remarks:<br>•This application was filed on 03-05-2012 as a divisional application to the application mentioned under INID code 62.<br>•Claims filed after the date of filing of the application (Rule 68(4) EPC). |
| (72) Inventors:<br>• **Lockhart, Joseph**<br>**Raleigh, NC 27613 (US)** | |

(54) **Balsalazide formulations and manufacture thereof**

(57)    The invention relates to formulations and dosage schedules of balsalazide. The invention further relates to methods of producing pharmaceutical formulations of balsalazide.

**Description**

BACKGROUND

Balsalazide is a non-steroidal, anti-inflammatory aminosalicylate derivative which is useful in the treatment of gastrointestinal diseases, for example active ulcerative colitis, colon cancer, and Crohn's disease. See, for example WO 95/18622, US 6,197,341 and US 6,326,364, which are incorporated herein by reference in their entirety.

One disadvantage of balsalazide is that relatively high doses are required making it difficult to administer as a single dose. Balsalazide is also highly colored and thus its administration as a solution is disadvantageous because it stains the mouth. For compliance reasons the number of capsules to be swallowed by a patient per day should be as small as possible. Currently, balsalazide formulated as a capsule is large and is difficult in some cases to swallow.

Currently, balsalazide is administered as three capsules three times per day. Thus, subject compliance is a problem. There is a need in the art for more convenient dosing of balsalazide and a need to reduce the number of times per day of dosing. Thus, there is also a need in the art for new methods of making balsalazide to accommodate the more convenient dosing schedules.

SUMMARY

Described herein are novel methods of making and dosage schedules of balsalazide, that meet the current needs of the industry for more convenient dosing, which also reduce the amount of balsalazide necessary to treat the subject.

Presented herein, according to one aspect are pharmaceutical preparations to treat gastrointestinal disease comprising two daily doses of about 6 grams per day of balsalazide or a pharmaceutically acceptable prodrug, salt, solvate, or clathrate thereof. According to another aspect, presented are pharmaceutical preparations to treat gastrointestinal disease comprising two daily doses of about 6.6 grams per day of balsalazide or a pharmaceutically acceptable prodrug, salt, solvate, or clathrate thereof In one embodiment, the two daily doses comprise about 3.3 grams each.

In another embodiment, the gastrointestinal disease is one or more of gastrointestinal bacterial infection or bacterial overgrowth, proctitis, or colon cancer.

According to another embodiment, the pharmaceutical preparation is in the form of an injectible fluid, an aerosol, a cream, a gel, a tablet, a capsule, a syrup or a transdermal patch.

The pharmaceutical preparations presented herein, according to one embodiment, may further comprise excipients (e.g., hypromellose, magnesium stearate, and Opadry II yellow). In a related embodiment, preparations contains hypromellose at from between about 1 to about 5 % of the total weight of the preparation. In another related embodiment, the preparation contains magnesium stearate at from between about 0.5 % and about 2.5% of the total weight of the preparation.

The pharmaceutical preparations presented herein, according to one embodiment, may further comprise one or more of titanium dioxide, polydextrose, triacetin, macrogol, D&C Yellow #10 Aluminum Lake, or FD&C Yellow #6 Aluminum Lake.

The pharmaceutical preparations presented herein, according to another embodiment, may further comprise a coating solution. In a related embodiment, the coating solution comprises hypromellose and hydroxyproply cellulose.

A package containing a pharmaceutical preparation to treat gastrointestinal disease comprising two daily doses of a balsalazide equivalent to about 6 grams per day. According to another aspect, presented herein are packages containing a pharmaceutical preparation to treat gastrointestinal disease comprising two daily doses of 3.3 grams each of balsalazide. In a related embodiment, the package may further comprise instructions for use to treat one or more of gastrointestinal disease.

According to one embodiment, the doses are in the form of an injectible fluid, an aerosol, a cream, a gel, a tablet, a capsule, a syrup or a transdermal patch.

Also presented here, according to one aspect are methods of treating gastrointestinal disease comprising administering to a subject in need thereof from between about 6 grams and about 6.7 grams per day of balsalazide in two daily doses.

In one embodiment, the two daily doses are about 3.3 grams each. In a related embodiment, the two daily doses comprise three tablets each. In another related embodiment, the three tablets comprise about 1100 mg of balsalazide each. In a related embodiment, the doses are in the form of an injectible fluid, an aerosol, a cream, a gel, a pill, a capsule, a syrup or a transdermal patch.

The method of treating may further comprise, according to one aspect, identifying the subject in need of treatment for one or more of a gastrointestinal disease or proctitis.

Presented herein, according to another aspect, are methods of manufacturing a capsule, comprising:

granulating the balsalazide disodium and one or more excipients to form granules;

sizing the granules;

blending the granules for about 20 minutes to form a powder blend; and

encapsulating the powder blend.

In one embodiment, the one or more excipients are combined with the balsalazide and may comprise one or more of colloidal silicon dioxide or magnesium sterate. In a related embodiment, the excipients comprise from between about 2 to about 3% of the granules by weight. In another embodiment, the sizing is with a 12 mesh screen. In another related embodiment, the blending is with a double cone blender.

The method of manufacturing may further comprise, according to one embodiment, polishing the encapsulated powder blend.

In one embodiment, from between about 700 mg and about 1200 mg of powder blend is encapsulated. In another embodiment, each capsule contains 772 mg of final blend.

Further presented herein, according to one aspect, are methods for dissolution testing of balsalazide capsules, comprising:

stirring a balsalazide capsule in dissolution medium;

filtering the solution to form a filtrate;

sampling the filtrate; and

diluting the filtrate.

In one embodiment, the dissolution medium comprises 50 mM phosphate buffer of about pH 6.8. In a related embodiment, the diluting the filtrate comprises diluting 2 mL in about 98 mL of dissolution medium. In another related embodiment, the stirring is at 50 rpm.

The method, according to another embodiment, may further comprise analyzing the diluted filtrate.

Provided herein according to one aspect are pharmaceutical preparations of balsalazide made by the process, comprising granulating a balsalazide disodium feed powder and one or more excipients to form granules; blending the granules for about 20 minutes to form a powder blend; and encapsulating the powder blend, wherein the two daily doses of about 6 grams per day of balsalazide or a pharmaceutically acceptable prodrug, salt, solvate, or clathrate thereof are administered.

Provided herein according to one aspect are pharmaceutical preparations ofbalsalazide made by the process granulating the balsalazide disodium and one or more excipients to form granules; blending the granules for about 20 minutes to form a powder blend; and encapsulating the powder blend, wherein the powder blend has an average particle diameter of from between about 300 to about 675 $\mu$m.

In one embodiment, the method further comprises sizing the granules before blending.

In another embodiment, the feed powder has an average particle diameter of from between about 8 to about 30 $\mu$m.

In one embodiment, the powder blend has an average particle diameter of from about 300 to about 675 $\mu$m.

In another embodiment, the powder blend has an specific surface area of from between about 3500 to about 8500 cm$^2$/ml.

In one embodiment, the powder blend has a hardness (retention) of from between about 85 to about 90 (60 Mesh).

In another embodiment, the powder blend has a Loose Bulk Density (LBD) of from between about 0.67 to about 0.69 g/cc.

In one embodiment, the balsalazide disodium feed powder has a compression 20 K of from between about 1.28 to about 1.47 g/cc.

In another embodiment, the balsalazide disodium feed powder has a Tapped Bulk Density (TBD) of from between about 0.57 to about 0.63 g/cc.

In one embodiment, the balsalazide disodium feed powder has a moisture of from between about 0.10% and about 0.15 %.

Hence, the present invention comprises the following embodiments:

1. A pharmaceutical preparation to treat gastrointestinal disease comprising two daily doses of about 6 grams per day of balsalazide or a pharmaceutically acceptable prodrug, salt, solvate, or clathrate thereof.

2. A pharmaceutical preparation to treat gastrointestinal disease comprising two daily doses of about 6.6 grams per day of balsalazide or a pharmaceutically acceptable prodrug, salt, solvate, or clathrate thereof.

3. The pharmaceutical preparation of clause 1 or 2, wherein two daily doses comprise between about 3 to about 3.3 grams each.

4. The pharmaceutical preparation of clause 1 or 2, wherein the gastrointestinal disease is one or more of gastrointestinal bacterial infection or bacterial overgrowth, proctitis, or colon cancer.

5. The pharmaceutical preparation of clause 1 or 2, wherein the pharmaceutical preparation is in the form of an injectible fluid, an aerosol, a cream, a gel, a tablet, a capsule, a syrup or a transdermal patch.

6. The pharmaceutical preparation of clause 1, further comprising one or more of a carrier, a lubricant or a colorant.

7. The pharmaceutical preparation of clause 6, wherein the carrier is hypromellose, calcium carbonate, calcium phosphate, dibasic calcium phosphate, tribasic calcium sulfate, calcium carboxymethylcellulose, cellulose, cellulose, dextrates, dextrin, dextrose, fructose, lactitol, lactose, magnesium carbonate, magnesium oxide, matitol, maltodextrins, maltose, polydextrose, sorbitol, starch, sucrose, sugar, and xylitol.

8. The pharmaceutical preparation of clause 6, wherein the lubricant is one or more of magnesium stearate, agar, calcium stearate, ethyl oleate, ethyl laureate, glycerin, glyceryl palmitostmate, hydrogenated vegetable oil, macrogol, magnesium oxide, mannitol, poloxamer, glycols, sodium benzoate, sodium lauryl sulfate, sodium stearyl, sorbitol, stearic acid, talc, triacetin, zinc stearate.

9. The pharmaceutical preparation of clause 6, wherein the colorant is one or more of Opadry II yellow, D&C Yellow #10 Aluminum Lake, or FD&C Yellow #6 Aluminum Lake.

10. The pharmaceutical preparation of clause 6, wherein the hypromellose is from between about 1 to about 5 % of the total weight of the preparation.

11. The pharmaceutical preparation of clause 6, wherein the magnesium stearate is from between about 0.5 % and about 2.5% of the total weight of the preparation.

12. The pharmaceutical preparation of clause 1, further comprising one or more of titanium dioxide, polydextrose, triacetin, macrogol, D&C Yellow #10 Aluminum Lake, or FD&C Yellow #6 Aluminum Lake.

13. The Pharmaceutical preparation of clause 1, further comprising a coating solution.

14. The pharmaceutical preparation of clause 13, wherein the coating solution comprises hypromellose and hydroxyproply cellulose.

15. A package containing a pharmaceutical preparation to treat gastrointestinal disease comprising two daily doses of a balsalazide equivalent to about 6 grams per day.

16. A package containing a pharmaceutical preparation to treat gastrointestinal disease comprising two daily doses of about 3.3 grams each of balsalazide.

17. The package of clause 16, further comprising instructions for use to treat one or more of gastrointestinal disease.

18. The package of clause 16, wherein the doses are in the form of an injectible fluid, an aerosol, a cream, a gel, a tablet, a capsule, a syrup or a transdermal patch.

19. A method of treating gastrointestinal disease comprising administering to a subject in need thereof from between about 6 grams and about 6.7 grams per day of balsalazide in two daily doses.

20. The method of clause 19, wherein the two daily doses are about 3.3 grams each.

21. The method of clause 19, wherein the two daily doses comprise three tablets each.

22. The method of clause21, wherein the three tablets comprise about 1100 mg of balsalazide each.

23. The method of clause 19, wherein the doses are in the form of an injectible fluid, an aerosol, a cream, a gel, a pill, a capsule, a syrup or a transdermal patch.

24. The method of clause 23, further comprising identifying the subject in need of treatment for one or more of a gastrointestinal disease or proctitis.

25. The method of clause 19, wherein the subject is a mammal.

26. The method of clause 19, wherein the subject is a human.

27. A method of manufacturing a capsule, comprising:

   granulating the balsalazide disodium and one or more excipients to form granules;
   sizing the granules;
   blending the granules for about 20 minutes to form a powder blend; and
   encapsulating the powder blend.

28. The method of clause 27, wherein the one or more excipients comprise one or more of colloidal silicon dioxide or magnesium stearate.

29. The method of clause 27, wherein the excipients comprise from between about 2 to about 3% of the granules by weight.

30. The method of clause 27, wherein the sizing is with a 12 mesh screen.

31. The method of clause 27, wherein the blending is with a double cone blender.

32. The method of clause 27, further comprising polishing the encapsulated powder blend.

33. The method of clause 27, wherein from between about 700 mg and about 1200 mg of powder blend is encapsulated.

34. A method for dissolution testing of balsalazide capsules, comprising:

   stirring a balsalazide capsule prepared according to claim 19 in dissolution medium;
   filtering the solution to form a filtrate;
   sampling the filtrate; and
   diluting the filtrate.

35. The method of clause 34, wherein the dissolution medium comprises 50 mM phosphate buffer of about pH 6.8.

36. The method of clause 34, wherein the diluting the filtrate comprises diluting 2 mL in about 98 mL of dissolution medium.

37. The method of clause 34, further comprising analyzing the diluted filtrate.

38. The method of clause 34, wherein the stirring is at 50 rpm.

39. A pharmaceutical preparation of balsalazide made by the process, comprising:

   granulating a balsalazide disodium feed powder and one or more excipients to form granules;
   blending the granules for about 20 minutes to form a powder blend; and encapsulating the powder blend,

wherein the two daily doses of about 6. grams per day of balsalazide or a pharmaceutically acceptable prodrug, salt, solvate, or clathrate thereof are administered.

40. The pharmaceutical preparation of clause 39, wherein the method further comprises sizing the granules before blending.

41. The method of clause 39, wherein the feed powder has an average particle diameter of from between about 8 to about 30 $\mu$m.

42. The method of clause 39, wherein the powder blend has an average particle diameter of from about 300 to about 675 $\mu$m.

43. The method of clause 39, wherein the powder blend has an specific surface area of from between about 3500 to about 8500 cm$^2$/ml.

44. The method of clause 39, wherein the powder blend has a hardness (retention) of from between about 85 to about 90 (60 Mesh).

45. The method of clause 39, wherein the powder blend has a Loose Bulk Density (LBD) of from between about 0.67 to about 0.69 g/cc.

46. The method of clause 39, wherein the balsalazide disodium feed powder has a compression 20 K of from between about 1.28 to about 1.47 g/cc.

47. The method of clause 39, wherein the balsalazide disodium feed powder has a Tapped Bulk Density (TBD) of from between about 0.57 to about 0.63 g/cc.

48. The method of clause 39, wherein the balsalazide disodium feed powder has a moisture of from between about 0.10% and about 0.15 %.

49. A pharmaceutical preparation of balsalazide made by the process, comprising:

   granulating the balsalazide disodium and one or more excipients to form granules;
   blending the granules for about 20 minutes to form a powder blend; and encapsulating the powder blend,

wherein the powder blend has an average particle diameter of from between about 300 to about 675 $\mu$m.

50. The pharmaceutical preparation of clause 49, wherein the method further comprises sizing the granules before blending.

51. The pharmaceutical preparation of clause 49, wherein the method further comprises sizing the granules before blending.

52. The method of clause 49, wherein the feed powder has an average particle diameter of from between about 8 to about 30 $\mu$m.

53. The method of clause 49, wherein the powder blend has an specific surface area of from between about 3500 to about 8500 cm$^2$/ml.

54. The method of clause 49, wherein the powder blend has a hardness (retention) of from between about 85 to about 90 (60 Mesh).

55. The method of clause 49, wherein the powder blend has a Loose Bulk Density (LBD) of from between about 0.67 to about 0.69 g/cc.

56. The method of clause 49, wherein the balsalazide disodium feed powder has a compression 20 K of from between about 1.28 to about 1.47 g/cc.

57. The method of clause 49, wherein the balsalazide disodium feed powder has a Tapped Bulk Density (TBD) of from between about 0.57 to about 0.63 g/cc.

58. The method of clause 49, wherein the balsalazide disodium feed powder has a moisture of from between about 0.10% and about 0.15 %.

**Other embodiments of the invention are disclosed *infra.***

BRIEF DESCRIPTION OF THE DRAWINGS

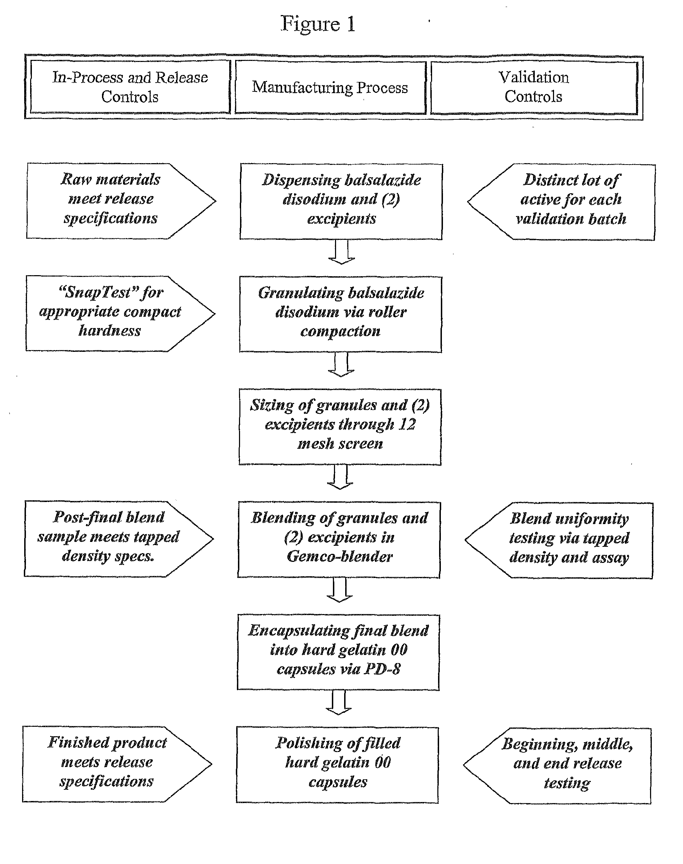

Figure 1 depicts a process flow diagram for the preparation of balsalazide capsules.

Figure 2 depicts a particle size distribution for one lot of drug substance (feed material)

Figure 3 depicts a particle size distribution for a second lot of drug substance (feed material)

Figure 4 depicts a particle size distribution for a third lot of drug substance (feed material)

Figure 5 depicts a particle size distribution for a fourth lot of drug substance (feed material)

Figure 6 depicts a comparison of particle size distributions from four lots of drug substance (feed material)

Figure 7 depicts a comparison of particle size distributions from three lots of drug substance (feed material)

Figure 8 depicts a comparison of particle size distributions from three lots of final blend of drug product using the three lots of drug substance (feed material) depicted in Figure 7.

DETAILED DESCRIPTION

The dosage regimen of balsalazide and formulations disclosed herein provides unexpected benefits that are of major significance for the subject. The new dosage formulations contain less balsalazide are safer than previously known formulations yet symptom relief, in particular reduction of abdominal pain, bloating and cramping, obtained with the regimen of the present invention is substantially equivalent to that for a regimen given three times/day and where the active principle level is higher. The present invention provides for more convenient dosing, reducing a significant barrier to patient compliance as measured by the adverse event occurrences. Another advantage of the present regimen over those given more often and with higher levels is that it offers an increased margin of safety because of its lower drug level. The new dosage formulations made it necessary to reformulate the methods of packaging balsalazide, thus presented herein are novel methods of manufacturing balsalazide capsules and tablets.

The new process or producing the balsalazide product is advantageous because it is able to utilize powder blends from more than one manufacture (e.g., API) having different particle sizes and blend to unexpectedly have similar dissolution profiles.

Balsalazide is a prodrug that is enzymatically cleaved in the colon to produce mesalainine (5-aminosalicylic acid), an antiinflammatory drug. Balsalazide disodium has the chemical name (E)-5-[[-4-[[(2-carboxyethyl) amino]carbonyl] phenyl]azo]-2-hydroxybenzoic acid, disodium salt, dihydrate. Its structural formula is $C_{17}H_{13}N_3O_6Na_2 \cdot 2H_2O$, having a molecular weight of 437.32.

Balsalazide disodium is a stable, odorless orange to yellow microcrystalline powder. It is freely soluble in water and isotonic saline, sparingly soluble in methanol and ethanol, and practically insoluble in all other organic solvents.

Balsalazide disodium is delivered intact to the colon where it is cleaved by bacterial azoreduction to release equimolar quantities of mesalamine, which is the therapeutically active portion of the molecule, and 4-aminobenzoyl-β-alanine. The current recommended dose of 6.75 grams/day, for the treatment of active disease, provides 2.4 grams of free 5- aminosalicylic acid to the colon. The formulations presented herein advantageously provide for administration of less balsalazide to a subject. Once administered, the 4-aminobenzoyl-β-alanine carrier moiety is released when balsalazide disodium is cleaved. The carrier is only minimally absorbed and is largely inert. The mechanism of action of 5-aminosalicylic acid is unknown, but appears to be topical rather than systemic. Mucosal production of arachidonic acid metabolites, both through the cyclooxygenase pathways, i.e., prostanoids, and through the lipoxygenase pathways, i.e., leukotrienes and hydroxyeicosatetraenoic acids, is increased in patients with chronic inflammatory bowel disease, and it is possible that 5-aminosalicylic acid diminishes inflammation by blocking production of arachidonic acid metabolites in the colon.

Upon reaching the colon, bacterial azoreductases cleave the compound to release 5-aminosalicylic acid, the therapeutically active portion of the molecule, and 4-aminobenzoyl-β-alanine.

As used herein, "treat, prevent or alleviate gastro intestinal disease" refers to the prophylactic use of the therapeutic agents described herein, e.g., balsalazide and the prophylactic use and use after diagnosis of gastrointestinal disease.

The term "subject" includes organisms which are capable of suffering from gastrointestinal disease or who could otherwise benefit from the administration of a composition of the invention, such as human and non-human animals. Preferred human animals include human patients suffering from or prone to suffering from a gastrointestinal disease or associated state, as described herein. The term "non-human animals" of the invention includes all vertebrates, e.g., mammals, e.g., rodents, e.g., mice, and non-mammals, such as non-human primates, e.g., sheep, dog, cow, chickens, amphibians, reptiles, *etc.* The preparations are also useful for veterinary purposes. A composition comprising a 5-aminosalicylate compound described herein, can be administered to a non-human vertebrate including, but not limited to, a wild, domestic or farm animal.

A method for "predicting or diagnosing" as used herein refers to a clinical or other assessment of the condition of a subject based on observation, testing, or circumstances.

"Therapeutically effective amount" as used herein refers to an amount of an agent which is effective, upon single or multiple dose administration to the cell or subject, in or in prolonging the survivability or comfort of the patient with such a disorder beyond that expected in the absence of such treatment. The term "effective amount" refers to a dosage or amount that is sufficient to reduce, alleviate or ameliorate the symptoms of gastrointestinal disease in a subject or to achieve a desired biological outcome as measured in the diagnostic tests described herein.

As used herein, "gastrointestinal disorder" refers to and includes, for example, ulcerative colitis, Crohn's disease, irritable bowel syndrome, colon cancer, bacterial 5 infection, bacterial overgrowth, and/or proctitis (e.g., radiation induced proctitis).

As used herein, the term "treatment" is defined as the application or administration of a therapeutic agent to a subject or application or administration of a therapeutic agent to an isolated tissue or cell line from a subject, who has, or is at risk of having, 0 gastrointestinal disorder, with the purpose to cure, heal, alleviate, relieve, alter, remedy, ameliorate, improve or affect the symptoms.

## Pharmaceutical Preparation

Pharmaceutical preparations of balsalazide are described herein. The formulations are 5 suitable to treat gastrointestinal disorders, e.g., bacterial infection or bacterial overgrowth, suitable non-systemic delivery routes include, for example, an ingestive delivery route or a colonic delivery route. A preferred delivery route is an ingestive delivery route, whereby the balsalazide enters the gastrointestinal or digestive tract by way of voluntary or forced ingestion through the mouth. The organs of a gastrointestinal tract include the esophagus, stomach, large intestine, small intestine, and rectum. The skilled artisan will be aware that in a non-human vertebrate the digestive tract may include a rumen, crop, gullet, cecum, or other specialized organ as pertains to a particular vertebrate species.

Dosage formes that are suitable for oral administration can be coated to reduce or avoid degradation of the active ingredient within the gastrointestinal tract.

Pharmaceutical preparations, in the form of, for example, tablets, caplets, and capsules may contain from about 100 mg to about 1.400 mg of the pharmaceutical composition (i.e., balsalazide and excipient(s)), more preferably from about 700 mg to about 1200 mg of the composition. Specific single unit dosage forms of the invention contain 50, 100, 150, 200, 250, 300 , 350, 400, 500, 750, 1000, 1100, 1200, 1300, 2000, 2500, 3000, or 3300 mg of active ingredient. Capsules can be of any size. Examples of standard sizes include #000, #00, #0, #1, #2, #3, #4, and #5. See, e.g., Remington's Pharmaceutical Sciences, page 1658-1659 (Alfonso Gennaro ed., Mack Publishing Company, Easton Pa., 18th ed., 1990). Preferred capsules of the invention are of size #00, #2, or #4.

Pharmaceutical compositions and dosage forms of the invention preferably contain one or more excipients in an amount of less than about 75 percent by weight of the total composition or dosage form. Pharmaceutical compositions and dosage forms are encompassed by the invention that contain the excipient(s) in an amount of from about 0.1 percent to about 60 percent by weight, preferably from about 0.5 percent to about 10 percent by weight, more preferably in an amount of about 3 percent by weight.

Excipients include carriers, diluents, fillers, lubricants, glidants, wetting, emulsifying, coloring agents, and pH buffering agents. One embodiment of the invention encompasses a pharmaceutical composition that includes balsalazide, and a carrier, diluent or filler. The carrier, diluent or filler is preferably present in an amount from about 0.1 percent to about 30 percent by weight, preferably from about 1 percent to about 5 percent by weight. A preferred pharmaceutical composition further includes a lubricant or glidant in an amount of from about 0.01 percent to about 4 percent by weight, and more preferably in an amount from about 0.1 percent to about 1 percent. In yet another embodiment, the composition further includes a disintegrant, preferably in an amount from about 1 percent to about 8 weight percent, more preferably from about 1 percent to about 3 weight percent.

Carriers, diluents and fillers suitable for use in pharmaceutical compositions and dosage forms include, but are not limited to, calcium carbonate, calcium phosphate, dibasic calcium phosphate, tribasic calcium sulfate, calcium carboxymethylcellulose, cellulose, cellulose (e.g., microcrystalline cellulose, silicified microcrystalline cellulose, and cellulose acetate), dextrates, dextrin, dextrose (glucose), fructose, lactitol, lactose, magnesium carbonate, magnesium oxide, hypromellose, matitol, maltodextrins, maltose, polydextrose, sorbitol, starch (e.g., pregelatinized starch), sucrose, sugar, and xylitol. One example of a pre-gelatinized starch is SPRESS B-820. Suitable forms of microcrystalline cellulose include, but are not limited to, the materials sold as AVICEL-PH-101, AVICEL-PH-103 AVICEL RC-581, AVICEL-PH-105 (available from FMC Corporation, American Viscose Division, Avicel Sales, Marcus Hook, Pa.), PROSOLV SMCC 90HD (Penwest, Patterson, N.Y.), and mixtures thereof. Carriers, diluents and fillers may also be used in premixes. Lubricants that can be used in pharmaceutical compositions and dosage forms of the invention include, but are not limited to, agar, calcium stearate, ethyl oleate, ethyl laureate, glycerin, glyceryl palmitostearate, hydrogenated vegetable oil (e.g., corn oil, cottonseed oil, olive oil, peanut oil, sesame oil, soybean oil, and sunflower oil), macrogol, magnesium oxide, magnesium stearate, mannitol, poloxamer, glycols (e.g., polyethylene glycol), sodium benzoate, sodium lauryl sulfate, sodium stearyl, sorbitol, stearic acid, talc, triacetin, zinc stearate, and mixtures thereof. Glidants include, for example, colliodal silicon dioxide, coagulated aerosols of synthetic silica colloidal silicon dioxide, magnesium trisilicate, powdered cellulose, pyrogenic silicon dioxide products (e.g., CAB-O-SIL sold by Cabot Co. of Boston, Mass.), starch, syloid silica gels (e.g., AEROSIL 200, manufactured by W.R. Grace Co. of Baltimore, Md.), talc, tribasic calcium phosphate, and mixtures thereof. If used, lubricants are typically used in an amount of less than about 1 weight percent of the pharmaceutical compositions or dosage forms into which they are incorporated.

Colorants may include, for example, D&C Yellow #10 Aluminum Lake, and/or FD&C Yellow #6 Aluminum Lake.

Disintegrants may be used in the compositions to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form the compositions of the invention. The amount of disintegrant used varies based upon the type of formulation, and is readily discernibly to those of ordinary skill in the art. Disintegrants that can be used in pharmaceutical compositions and dosage forms of the invention include, but are not limited to, agar-agar, algins (e.g., alginic acid), calcium carbonate, carboxymethylcellulose, cellulose (e.g., hydroxypropyl cellulose, microcrystalline cellulose, and silicified microcrystalline cellulose), clays, colloidal silicon dioxide, croscarmellose sodium, crospovidone, gums, magnesium aluminium silicate, methylcellulose, polacrilin potassium, sodium alginate, sodium starch glycolate, starch (e.g., pregelatinized starch, potato starch, and tapioca starch), and mixtures thereof.

Pharmaceutical compositions of the invention suitable for administration can be presented as discrete dosage forms, such as capsules (e.g., gelcaps), caplets, tablets, troches, lozenges, dispersions, and suppositories each containing a predetermined amount of an active ingredient as a powder or in granules, a solution, or a suspension in an aqueous or non-aqueous liquid, an oil-in-water emulsion, or a water-in-oil liquid emulsion. Because of their ease of administration, tablets, caplets, and capsules represent a preferred oral dosage unit forms.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions that can be used include polymeric substances and waxes. The active ingredient can also be in micro-encapsulated form, if appropriate, with one or more of the above-described excipients.

Presented herein in an alternative tablet dosage form for balsalazide disodium that contains 1.1 g of balsalazide disodium per tablet compared to the currently approved dosage strength/form of 750 mg/capsule. The NDA for Colazal (balsalazide disodium) Capsules (NDA 20-610) was approved by the FDA on July 18, 2000 for the treatment of mildly to moderately active ulcerative colitis using a dosage and administration regimen of three 750 mg Colazal Capsules three times daily (6.75 g/day) for 8 weeks. Presentedherein is a dosing regimen of three balsalazide tablets (1.1 g/tablet) twice daily (6.6 g/day). The treatment period may range from 1 day to 15 weeks, 1 to 9 weeks, or for example, 6 weeks.

Efficacy of treatment, for example, for ulcerative colitis may be measured by the rectal bleeding subscale of the Modified Mayo Disease Activity index (MMDAI), where clinical improvement is defined as a 3 point reduction from baseline in the MMDAI. In addition, a single dose food-effect study may also be used to validate the efficacy.

The unit formulation according to the invention is preferably provided with a coating, preferably a saliva resistant, optionally enteric, coating. The coating preferably comprises from 4 to 8% by weight of the unit formulation, more preferably about 6%. The coating is preferably a film coating comprising a polymer (for example, hydroxypropylmethylcellulose, methyl cellulose, polymethylacrylate (for example Eudragit E, Eudragit L or Eudragit S or ethylcellulose), a plasticiser (for example PEG, propylene glycol, glycerol and its esters or a phthalate ester) and/or a colourant, e.g., water insoluble pigments.

Preferred pharmaceutical preparations include two daily doses of about 6 grams per day of balsalazide or a pharmaceutically acceptable prodrug, salt, solvate, or clathrate thereof. Further preferred dosage forms include two daily doses of about 6.6 grams per day of balsalazide or a pharmaceutically acceptable prodrug, salt, solvate, or clathrate thereof, wherein the two daily doses comprise about 3.3 grams each. The two daily doses may be given, for example, as three tablets.

In addition to the balsalazide, formulations provided herein may further comprise one or more of hypromellose, magnesium stearate, and Opadry II yellow. The hypromellose may be from between about 1 to about 5 % of the total weight of the preparation and the magnesium stearate may be from between about 0.5 % and about 2.5% of the total weight of the preparation. The formulations may further comprise one or more of titanium dioxide, polydextrose, triacetin, macrogol, D&C Yellow #10 Aluminum Lake, or FD&C Yellow #6 Aluminum Lake as well as a coating solution (e.g., hypromellose and hydroxyproply cellulose).

Table 1 presents an exemplary balsalazide formulation:

| Component | mg/tablet |
|---|---|
| Balsalazide disodium, dehydrate | 1100 |
| Hypromellose | 36 |
| Magnesium Stearate (Vegetable Source) | 17 |
| Opadry II Yellow (#Y-22-12553) | 35 |
| Hypromellose | |
| Titanium Dioxide | |
| Polydextrose | |
| Triacetin | |
| Macrogol | |
| D&C Yellow #10 Aluminum Lake | |
| FD&C Yellow #6 Aluminum Lake | |
| Coating solution | 4.3 |
| Hypromellose | |
| Hydroxypropyl Cellulose | |

Pharmaceutical compositions and dosage forms of the invention may also contain one or more secondary active ingredients or may be co-administered with the secondary active ingredients. Examples of secondary active ingredients include, but are not limited to, anti-cancer drugs, anti-inflammatory (steroid or a non-steroidal agents), and/or anti-nausea.

Useful non-steroidal anti-inflammatory agents, include, but are not limited to, aspirin, ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, amigloprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflurnic acid, tolfenamic acid, diflurisal, flufenisal, piroxicam, sudoxicam, isoxicam; salicylic acid derivatives, including aspirin, sodium salicylate, choline magnesium trisalicylate, salsalate, diflunisal, salicylsalicylic acid, sulfasalazine, and olsalazin; para-aminophennol derivatives including acetaminophen and phenacetin; indole and indene acetic acids, including indomethacin, sulindac, and etodolac; heteroaryl acetic acids, including tolmetin, diclofenac, and ketorolac; anthranilic acids (fenamates), including mefenamic acid, and meclofenamic acid; enolic acids, including oxicams (piroxicam, tenoxicam), and pyrazolidinediones (phenylbutazone, oxyphenthartazone); and alkanones, including nabumetone and pharmaceutically acceptable salts thereof and mixtures thereof. For a more detailed description of the NSAIDs, *see* Paul A. Insel, Analgesic-Antipyretic and Antiinflammatory Agents and Drugs Employed in the Treatment of Gout, in Goodman & Gilman's The Pharmacological Basis of Therapeutics 617-57 (Perry B. Molinhoff and Raymond W. Ruddon eds., 9th ed 1996) and Glen R. Hanson, Analgesic, Antipyretic and Anti-Inflammatory Drugs in Remington: The Science and Practice of Pharmacy Vol II 1196-1221 (A.R. Gennaro ed. 19th ed. 1995) which are hereby incorporated by reference in their entireties.

Other secondary active ingredients may include, but are not limited to, immunomodulatory agents, anti-inilammatory agents (*e.g.*, adrenocorticoids, corticosteroids (*e.g.*, beclomethasone, budesonide, flunisolide, fluticasone, triamcinolone, methlyprednisolone, prednisolone, prednisone, hydrocortisone), glucocorticoids, steroids, non-steriodal anti-inflammatory drugs (*e.g.*, aspirin, ibuprofen, diclofenac, and COX-2 inhibitors), and leukotreine antagonists (*e.g.*, montelukast, methyl xanthines, zafirlukast, and zileuton), beta2-agonists (*e.g.*, albuterol, biterol, fenoterol, isoetharie, metaproterenol, pirbuterol, salbutamol, terbutalin formoterol, salmeterol, and salbutamol terbutaline), anticholinergic agents (*e.g.*, ipratropium bromide and oxitropium bromide), sulphasalazine, penicillamine, dapsone, antihistamines, anti-malarial agents (*e.g.*, hydroxychloroquine), anti-viral agents, and antibiotics (*e.g.*, dactinomycin (formerly actinomycin), bleomycin, erythomycin, penicillin, mithramycin, anthramycin (AMC)), and anti-cancer drugs.

Examples of anti-cancer drugs include, for example, acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; duazomycin; edatrexate; eflomithine hydrochloride; elsamitrucin; enloplatin; enpromate; epipropidine; epirabicin hydrochloride; erbulozole; gemcitabine; gemcitabine hydrochloride; hydroxyurea; idarubicin hydrochloride; ifosfamide; ilmofosine; interleukin II (including recombinant interleukin II, or rIL2), interferon alfa-2a; interferon alfa-2b; interferon alfa-n1; interferon alfa-n3; interferon beta-I a; interferon gamma-I b; iproplatin; irinotecan hydrochloride; lanreotide acetate; letrozole; leuprolide acetate; liarozole hydrochloride; lometrexol sodium; lomustine; losoxantrone hydrochloride; masoprocol; menogaril; mercaptopurine; methotrexate; methotrexate sodium; metoprine; meturedepa; mitindomide; mitocarcin; mitocromin; mitogillin; mitomalcin; mitomycin; mitosper; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; and zinostatin stimalamer.

In combination therapy treatment, both the compounds of this invention and the other drug agent(s) are administered to mammals (*e.g.*, humans, male or female) by conventional methods. The agents may be administered in a single dosage form or in separate dosage forms. Effective amounts of the other therapeutic agents are well known to those skilled in the art. However, it is well within the skilled artisan's purview to determine the other therapeutic agent's optimal effective-amount range. In one embodiment of the invention where another therapeutic agent is administered to an animal, the effective amount of the compound of this invention is less than its effective amount would be where the other therapeutic agent is not administered. In another embodiment, the effective amount of the conventional agent is less than its effective amount would be where the compound of this invention is not administered. In this way, undesired side effects associated with high doses of either agent may be minimized. Other potential advantages (including without limitation improved dosing regimens and/or reduced drug cost) will be apparent to those of skill in the art.

Pharmaceutical packs or kits which comprise pharmaceutical compositions or dosage forms disclosed herein are also encompassed by the present invention. An example of a kit comprises notice in the form prescribed by a governmental agency regulating the manufacture, use or sale of pharmaceuticals or biological products, which notice reflects approval by the agency of manufacture, use or sale for human administration.

A package containing a pharmaceutical preparation to treat gastrointestinal disease may comprise, for example, two daily doses of a balsalazide equivalent to about 6 grams to about 6.7 grams per day, preferably, about 6.6 grams. The packages may contain for example, two daily doses of 3.3 grams each of balsalazide.

**Methods of Treatment**

Methods of treating gastrointestinal diseases presented herein are advantageous over the previously known methods. The methods of treatment may be used in a prophylactic manner or after diagnosis. Methods of diagnosis (identification of a subject in need of treatment) are discussed and monitoring treatment is also discussed below.

The invention also encompasses a method of reducing or preventing an adverse effect associated with chemotherapy or radiation therapy, which comprises administering to a patient in need of such treatment or prevention a pharmaceutical composition or dosage form of the invention in an amount sufficient to reduce an adverse effect associated with the chemotherapy or radiation therapy. This embodiment includes the use of pharmaceutical compositions and dosage forms to protect against or treat an adverse effect associated with the use of chemotherapy or radiation therapy, including raising a subject's tolerance for chemotherapy or radiation therapy.

Identification of a subj ect for treatment, diagnosis, and monitoring of treatment may be measured by methods that are well known to the skilled artisan, for example by the measurement of bacterial growth in the intestinal tract. Many fermentative bacterial species found in the gastrointestinal tract produce detectable quantities of hydrogen or methane gas in the presence of certain sugars, which gases enter the blood stream of the host and are exhaled. This is the basis for intestinal bacterial growth detection means, such as, but not limited to, the lactulose, glucose, or lactose breath hydrogen tests (e.g., P. Kerlin and L. Wong, Breath hydrogen testing in bacterial overgrowth of the small intestine, Gastroenterol. 95(4):982-88 [1988]; A. Strocchi et al., Detection of malabsorption of low doses of carbohydrate: accuracy of various breath H2 criteria, Gastroenterol. 105(5):1404-1410 [1993]).

Alternatively, bacterial growth in a gastrointestinal tract is measured by detection of $^{13}CO_2$ or $^{14}CO_2$ breath emissions after administering an isotope-labeled sugar that is metabolizable by gastrointestinal bacteria but non-digestible by the host, such as, but not limited to, xylose or lactulose in humans. (E.g., G. R. Swart and J. W. van den Berg, 13C breath test in gastrointestinal practice, Scand. J. Gastroenterol. [Suppl.] 225:13-18 [1998]; C. E. King and P. P. Toskes, Breath tests in the diagnosis of small intestinal bacterial overgrowth, Crit. Rev. Lab. Sci. 21(3):269-81 [1984]; C. S. Chang et al., Increased accuracy of the carbon-14D-xylose breath test in detecting small-intestinal bacterial overgrowth by correction with the gastric emptying rate, Eur. J. Nucl. Med. 22(10):1118-22 [1995]; A. Schneider et al., Value of the 14C-D-xylose breath test in patients with intestinal bacterial overgrowth, Digestion 32(2):86-91 [1985]).

Direct gastrointestinal sampling or biopsy from any body site or tissue can also be used to measure the inhibition of bacterial growth in a gastrointestinal tract or other body site or tissue. As the skilled artisan is aware, direct sampling at time intervals provides information about the growth inhibition of specific bacterial species of interest, to which breath testing is not well-suited. Samples are diluted and bacterial numbers can be assessed by conventional microbiological means such as, but not limited to colony plating or Most Probable Number (MPN) techniques, or direct counting of bacterial cells. For direct bacterial cell counts, cells can optionally be labeled with specific markers, and counts can be accomplished manually or by devices such as fluorescence activated cell sorting (FACS).

Alternatively, evidence of inhibition of bacterial growth can be inferred by the practitioner treating a bacterial infection or intestinal bacterial overgrowth in a human or nonhuman vertebrate subject with observation of an improvement in various infection-or overgrowth-related symptoms in response to the administration of an antimicrobial composition of the present invention.

Among the bacterial species inhibited in accordance with the present inventive method are obligate anaerobes such as, but not limited to, Clostridium species. It is a particular advantage of the present invention that 5-aminosalicylic acid is an antimicrobial agent that does not affect many beneficial or commensal gastrointestinal bacteria but selectively inhibits potentially pathogenic clostridial species, such as, but not limited to, C. perfringens, C. difficile, C. tetani and C. botulinum.

Subjects may also be self-identified or identified by a treating health care professional based on symptoms.

Methods of treating gastrointestinal disease provided herein may comprise administering to a subject in need thereof from between about 6 grams and about 6.7 grams per day of balsalazide in two daily doses. The two daily doses may be, for example, about 3.3 grams each. The two daily doses may comprise three tablets each, wherein the three tablets comprise about 1100 mg ofbalsalazide each. The two daily doses may be taken with or without food or liquid and may be taken about 1 to about 23 hours, preferably from between about 4 and about 12 hours apart.

**Methods of Making Balsalazide Tablets**

Presented below are exemplary methods of making a balsalazide tablet:

**Initial Weighing**

Appropriate quantities of balsalazide disodium, and any appropriate excipients, e.g., hypromellose, and magnesium stearate, are dispensed.

**Wet Granulation**

Balsalazide Disodium and Hypromellose are granulated using a low shear (Planetary) mixer. The wet granules are tray dried in an oven to a moisture level of bout NMT 2.0%.

**Milling**

The dried granules are milled through a Fitzpatrick mill fitted, for example, with a #2AA mesh stainless steel screen, knives forward at medium speed.

**Final Blend**

After milling, the compacted granules are charged into a V-blender and blended with the Magnesium Stearate.

**Compression**

Using an automated tablet press, the final mixture is compressed into 1100 mg tablets.

**Tablet Coating**

The compressed tablets are coated.

**Packaging**

Balsalazide tablets are packaged into foil blister units or HDPE bottles.

**Methods of Making Balsalazide Capsules**

Presented below are exemplary methods of making a balsalazide capsule:

A 750 mg Balsalazide Disodium Capsule is an immediate release drug product. The manufacturing process for balsalazide capsules comprise roller compaction, oscillation, and blending of ingredients to produce a final blend that can be encapsulated within a defined fill weight range thereby producing a uniform distribution of balsalazide disodium.

Balsalazide capsules described herein comprise active Pharmaceutical ingredient, balsalazide disodium, and (2) excipients. The excipients comprise from between about 1% to about 5%, and preferably approximately 2.54% of the formulation. Colloidal silicon dioxide is used to reduce inter-particular adhesions and improve fluidity; magnesium stearate is added partially as a lubricant and as an aid in powder flow for encapsulation. Once the balsalazide disodium is roller compacted and oscillated, it is combined with the excipients in the final blend which completes the compounding process. This step-wise approach as described below ensures adequate, even distribution of the active ingredient in the final blend prior to encapsulation into hard gelatin 00 capsules. The process includes granulating the balsalazide disodium and one or more excipients to form granules; sizing the granules; blending the granules for about 20 minutes to form a powder blend; and encapsulating the powder blend. The encapsulated powder blend may optionally be polished after encapsulation. Suitable capsule polishers include, for example, an Acta capsule polisher.

**Dispensing**

Balsalazide disodium and excipients (e.g., colloidal silicon dioxide and magnesium stearate) are weighed out and transferred to the compounding area(s).

**Granulation**

The balsalazide disodium is granulated via roller compaction. The balsalazide disodium is force fed by means of a horizontal auger into two horizontally opposed roller drums. This compaction process transform the drug substance into ribbons of compacted granules of balsalazide disodium. The granulation maybe done, for example, by a Fitzpatrick Roller Compactor.

## Sizing

The ribbons of compacted granules of balsalazide disodium are size-reduced via an oscillator utilizing, for example, a 12 mesh stainless steel screen to give granules of uniform size. The colloidal silicon dioxide and magnesium stearate are also screened via a 12 mesh stainless steel screen. Other suitable screen include, an 11 mesh screen and a 13 mesh screen. Suitable oscillators for sizing include, for example, the Colton Oscillator #1 with a #12 mesh screen.

## Blending

The previously screened colloidal silicon dioxide, magnesium stearate, and the granules of balsalazide disodium are transferred to a double-cone blender and blended for between about 15 minutes and 20 minutes. Suitable benders include, for example, a Gemco double cone blender.

## Encapsulation

The powder blend is encapsulated into hard gelatin 00 capsules using a semi-automated or fully automated encapsulator. The filled capsules are then polished prior to dispensing into bulk containers. A suitable semi-automated encapsulator includes, for example, a Parke Davis Type 8. A suitable fully automated encapsulator includes, for example, a Bosch 1500.

In the formulations, described herein from between about 700 mg and about 1200 mg of powder blend is encapsulated or compressed. Preferable about 750 mg to about 1100 mg for tablets and/or capsules.

Table 2 below summarizes exemplary operating conditions for the manufacture of a balsalazide capsule according to the novel processed described herein.

| Operating Conditions | | | |
|---|---|---|---|
| Operating Parameter | Previously Validated Operating Range | Target Setting for Current Validation Batches | Actual Range for Current Validation Batches |
| Air Pressure | 30 psi | 30 psi | 30 psi |
| Roll Pressure | 800 kp | 800 kp | 800 kp |
| Roll Speed | 8-15 rpm | 8 - 15 rpm | 12.1 - 14.0 rpm |
| Horizontal Screw Speed | 17 - 31 rpm | 17 - 30 rpm | 26 - 30 rpm |
| Vertical Screw Speed | 300 - 400 rpm | 300 - 400 rpm | 365 - 375 rpm |
| Roll Gap | 0.02 - 0.03 at set-up | Monitor | 0.070 - 0.203* |
| Oscillation Screen | #12 mesh | #12 mesh | # 12 mesh |
| Blend Time | 28-32 minutes | 20 minutes | 20 minutes |
| Blend Speed | 12 rpm | 12 rpm | 12 rpm |
| Encapsulator Ring Size | N/A | "00" ring size | "00" ring size |
| Encapsulator Speed | 10 rpm | 10 rpm | 10 rpm |
| Polisher Speed | 5-9 rpm | 5-9 rpm | 7 rmp |
| * Roll Gap range monitored during batch processing (30 minute checks) | | | |

Table 3 below summarizes exemplary in-process blend validation results for capsule manufacturing according to the processed described herein.

| Test | Specification | Test Results | |
|---|---|---|---|
| Bulk density | Record Information | Top | 0.57 g/ml |
| | | Middle | 0.60 g/ml |
| | | Bottom | 0.60 g/ml |

(continued)

| Test | Specification | Test Results | |
|---|---|---|---|
| Tapped Density | 0.83-0.93 g/ml | Top | 0.86 g/ml |
| | | Middle | 0.86 g/ml |
| | | Bottom | 0.86 g/ml |
| Blend Assay ** | 95.0-105.0% RSD NMT 6.0 | 96-101% | RSD = 1.4 |

Table 4 below summarizes exemplary finished product validation results for capsule manufacturing according to the processed described herein.

| Test | Specification | Test Results | |
|---|---|---|---|
| Capsule Weight | 870mg-924mg | Beg | 892 mg |
| | | Middle | 891 mg |
| | | End | 875 mg |
| Assay | 95.0-105.0% | Beg | 100% |
| | | Middle | 99% |
| | | End | 97% |
| Weight Variation (range of 10 capsules) | 85-115% Per USP | Beg | 98-103% |
| | | Middle | 95-105% |
| | | End | 95-104% |
| Dissolution (average of 6 capsules) | NLT Q + 5% at 30 minutes where Q = 70% | Beg | 101% |
| | | Middle | 100% |
| | | End | 98% |

**Dissolution Testing**

Also presented herein are novel methods for dissolution testing of balsalazide formulations. The methods are further shown below in the examples.

Methods for dissolution testing of balsalazide capsules, comprise stirring a balsalazide capsule prepared according to the methods described herein in dissolution medium; filtering the solution to form a filtrate; sampling the filtrate; and diluting the filtrate.

The dissolution medium may comprise from between about 40 to about 60 mM phosphate buffer of between about pH 6.2 and pH 7. Preferable the dissolution buffer is 50 mM phosphate buffer of about pH 6.8.

The diluting the filtrate may comprise diluting about 0.5 mL to about 4 mL in about 25 mL to about 198 mL of dissolution medium.

Analyzing the diluted filtrate may be by coloumetric methods, UV methods, or by other methods known to those of skill in the art. One preferred method is by UV spectrophotometer at 352 mm.

The stirring of the balsalazide may be at from between about 25 to about 75 rpm. Preferably, the stirring is at about 50 rpm.

These new dissolution test methods are advantageous because they allow for higher discrimination.

EXAMPLES

It should be appreciated that the invention should not be construed to be limited to the examples which are now described; rather, the invention should be construed to include any and all applications provided herein and all equivalent variations within the skill of the ordinary artisan.

EXAMPLE 1

**Dissolution testing of Colazal Capsules, 750 mg**

**Materials**

High Purity Water (18 MΩ resistivity or greater)
Balsalazide Disodium Reference Standard
Monobasic Potassium Phosphate, ACS reagent grade
10N Sodium Hydroxide
Type 316 Stainless Steel Spiral Wire Cage, Quality Lab Accessories, Cat Number: CAPWHT-4S
Gelman Nylon filter, 25 mm, 0.2 μm pore size, Part Number 4436T
Vankel 10-μm Full Flow Filter, Part Number 17-400

**Specifications (Reference Current USP Chapter <711>)**

| | |
|---|---|
| Stage 1 | Each dosage unit must not be less than 75% dissolved after 30 minutes (Q = 70%) |
| Stage 2 | The mean of 12 dosage units from Stage 1 and Stage 2 must not be less than 70% dissolved after 30 minutes. No individual dosage unit should be less than 55% dissolved after 30 minutes. |
| Stage 3 | The mean of 24 dosage units from Stages 1, 2, and 3 must not be less than 70% dissolved after 30 minutes. No more than two dosage units should be less than 55% dissolved after 30 minutes. No individual dosage unit should be less than 45% dissolved after 30 minutes. (Q = 70%) |

**Dissolution Medium Preparation (50 m*M* Phosphate Buffer, pH 6.8)**

For each liter of dissolution medium prepared, dissolve 6.8 grams of potassium phosphate, monobasic, in 1000 mL of high purity water and mix well. Adjust the pH to 6.8 ± 0.05 with 10 *N* sodium hydroxide. Degas the dissolution medium via helium sparge for approximately 15 minutes for each six liters of medium prepared.

**Balsalazide Disodium Stock Standard Preparation (0.175 mg/mL)**

Prepare the stock standard solution in duplicate. For each preparation, transfer approximately 35 mg of Balsalazide Disodium Reference Standard, accurately weighed, to a 200-mL volumetric flask. Add 150 mL of dissolution medium, swirl and briefly sonicate to dissolve the flask contents. Dilute to volume with dissolution medium and mix well. Stock Standard Preparation A should be used for analysis of dissolution samples. Prep B should be used as the standard check preparation. The stock standard preparation is stable for 4 days when stored at room temperature and unprotected from light.

**Balsalazide Disodium Working Standard Preparation (0.0175 mg/mL)**

For each of the stock standard preparations, pipet 5.0 mL of the stock solution into a 50-mL volumetric flask, dilute to volume with dissolution medium, and mix well. The working standard preparation is stable for 4 days when stored at room temperature and unprotected from light.

**Sample Preparation**

Perform USP Apparatus 2 dissolution testing on six individual capsules utilizing the dissolution conditions listed below. Immediately, filter the samples using a 0.2 μm Gelman Nylon filter or a 10-μm Vankel Full Flow filter, discarding the first 2 mL of sample. Pipet 2.0 mL of the sample filtrate into a 100-mL volumetric flask, dilute to volume with dissolution medium, and mix well (nominal concentration: 0.017 mg Balsalazide Disodium per mL). Sample preparations are stable for 4 days when stored at room temperature and unprotected from light.

Dissolution Conditions

| Apparatus: | USP Rotating Paddles (Apparatus 2) |
| --- | --- |
| Sample Size: | Six single capsules with sinkers |
| Temperature: | 37°C±0.5°C |
| Stirring Speed: | 50 RPM |
| Medium: | 900 mL |
| Pull Volume: | 10 mL |
| Sampling Times: | 10, 20, and 30 minutes |

**Procedure**

Using a suitable UV spectrophotometer at 352 nm and a 1.0-cm pathlength quartz cell, obtain a Dissolution Medium blank reading, five replicate standard readings, and two replicate standard check readings to demonstrate system suitability (see system suitability requirements below). Analyze the standard and samples in such a sequence that standard bracketing is used after every six Sample Preparation readings. Samples should only be read once. Calculate the percent Balsalazide Disodium dissolved (% LC) as shown below. Report the individual results and the mean to whole numbers. Report the % RSD to one decimal place.

**System Suitability Test**

The relative standard deviation (RSD) of Balsalazide Disodium absorbance readings throughout the analysis should not be more than 2.0 percent. The Balsalazide Disodium Standard and Standard Check Preparations must agree within 98.0 to 102.0%.

**Calculations**

Calculate the measured sample concentration ($C_i$) of Balsalazide Disodium at each timepoint ($n$):

$$Ci = \frac{Aspl}{Astd} \times \frac{Wstd \times Pstd}{Vstd} \times \frac{Dstd}{Dspl}$$

Where:

$A_{spl}$ = Absorbance of the sample solution (AU)
$A_{std}$ = Average absorbance of all standard readings throughout the analysis (AU)
$W_{std}$ = Weight of the standard (mg)
$P_{std}$ = Purity Factor of the standard as found on the certificate of analysis (decimal)\
$V_{std}$ = Volume of the stock standard solution (mL)
$D_{std}$ = Dilution factor of the working standard solution (mL/mL)
$D_{spl}$ = Dilution factor of the working sample solution (mL/mL)

Calculate the amount of Balsalazide Disodium dissolved at each timepoint:

$$\%Dissolved = \frac{\{C_i \times [V - V_r(n-1)]\} + \sum_{i=1}^{n-1} C_i \times V_r}{LC} \times 100$$

| Timepoint Number ($n$) | Timepoint (min) | $\sum_{i=1}^{n-1} C_i \times V_r$ |
|---|---|---|
| 1 | 10 | 0 |
| 2 | 20 | $C_1 \times V_r$ |
| 3 | 30 | $(C_1+C_2) \times V_r$ |

Where
$C_i$ = Concentration of the sample at timepoint n (mg/mL)
$V$ = Volume of the dissolution medium in the dissolution vessel at the start of the test (mL)
$V_r$ = Volume of dissolution medium removed at each timepoint during the sampling process (mL)
$n$ = Timepoint number
$LC$ = Label claim
100 = Conversion to percent
Note: Alternative means of calculating the results are allowed as long as the calculations are equivalent.

**EXAMPLE 2**

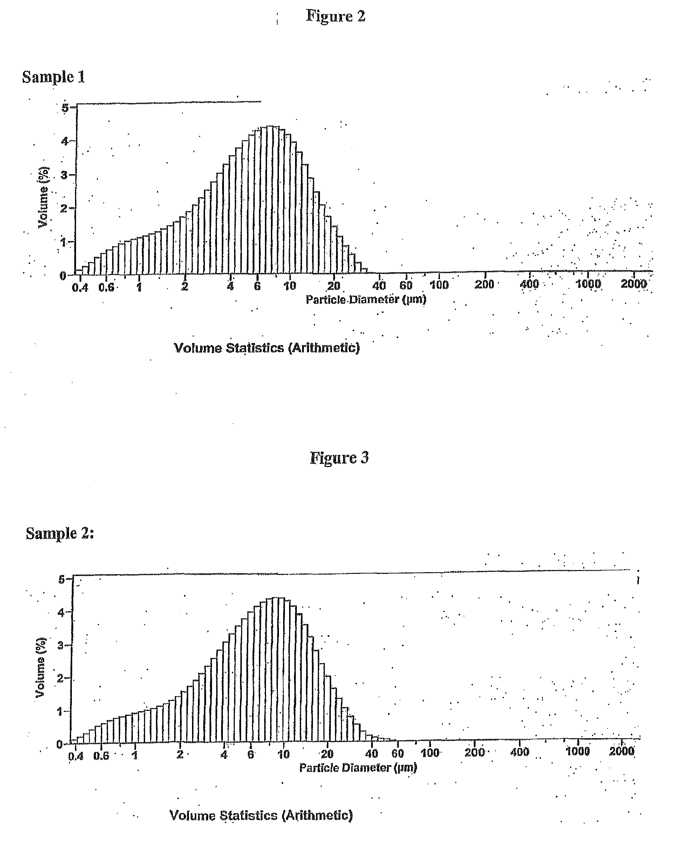

Figure 2 depicts and the data below tabulate for Sample 1 the arithmetic statistics describing surface area, particle size, and cumulative volume characteristics for one lot of drug substance (feed material). The particle size distribution for this lot is depicted in Figure 2.

**Sample 1:**

Optical model: Fraunhofer.rfz
LS 200          Small Volume Module

Calculations from 0.375 $\mu$m to 2000 $\mu$m

Volume:          100%
Mean:            6.967 $\mu$m
Median:          5.584 $\mu$m
Specific Surf. Area:   19389 cm$^2$/mL

| % < | 10 | 25 | 50 | 75 | 100 |
|---|---|---|---|---|---|
| $\mu$m | 1.266 | 2.827 | 5.584 | 9.624 | 43.67 |

**Table 5**

| Particle Diameter (Lower) $\mu$m | Cum. < Volume % | Diff. Volume % | Particle Diameter (Lower) $\mu$m | Cum. < Volume | Diff. Volume |
|---|---|---|---|---|---|
| 0.375 | 0 | 2.66 | 33.01 | 99.97 | 0.030 |

(continued)

| Particle Diameter (Lower) μm | Cum. < Volume % | Diff. Volume % | Particle Diameter (Lower) μm | Cum. < Volume | Diff. Volume |
|---|---|---|---|---|---|
| 0.656 | 2.66 | 6.09 | 57.77 | 100 | 0 |
| 1.149 | 8.74 | 8.50 | 101.1 | 100 | 0 |
| 2.011 | 17.2 | 14.4 | 176.9 | 100 | 0 |
| 3.519 | 31.6 | 22.8 | 309.6 | 100 | 0 |
| 6.158 | 54.5 | 25.3 | 541.9 | 100 | 0 |
| 10.78 | 79.8 | 15.8 | 948.3 | 100 | 0 |
| 18.86 | 95.6 | 4.34 | 1660 | 100 | 0 |

## EXAMPLE 3

Figure 3 depicts and the data below tabulate for Sample 2 the arithmetic statistics describing surface area, particle size, and cumulative volume characteristics for a second lot of drug substance (feed material). The particle size distribution for this lot is depicted in Figure 3.

**Sample 2:**

Optical model:    Fraunhofer.rfz
LS 200              Small Volume Module

Calculations from 0.375 μm to 2000 μm

Volume:           100%
Mean:             6.503 μm
Median:           5.044 μm
Specific Surf. Area:   20709 cm$^2$/mL

| % < | 10 | 25 | 50 | 75 | 100 |
|---|---|---|---|---|---|
| μm | 1.192 | 2.586 | 5.044 | 8.715 | 47.94 |

Table 6

| Particle Diameter (Lower) μm | Cum. < Volume % | Diff. Volume % | Particle Diamete (Lower) μm | Cum. < | Diff. |
|---|---|---|---|---|---|
| 0.375 | 0 | 2.91 | 33.01 | 99.8 | 0.23 |
| 0.656 | 2.91 | 6.59 | 57.77 | 100 | 0 |
| 1.149 | 9.50 | 9.37 | 101.1 | 100 | 0 |
| 2.011 | 18.9 | 16.1 | 176.9 | 100 | 0 |
| 3.519 | 35.0 | 24.3 | 309.6 | 100 | 0 |
| 6.158 | 59.3 | 24.1 | 541.9 | 100 | 0 |

(continued)

| Particle Diameter (Lower) μm | Cum. < Volume % | Diff. Volume % | Particle Diamete (Lower) μm | Cum. < | Diff. |
|---|---|---|---|---|---|
| 10.78 | 83.4 | 12.6 | 948.3 | 100 | 0 |
| 18.86 | 96.0 | 3.78 | 1660 | 100 | 0 |

## EXAMPLE 4

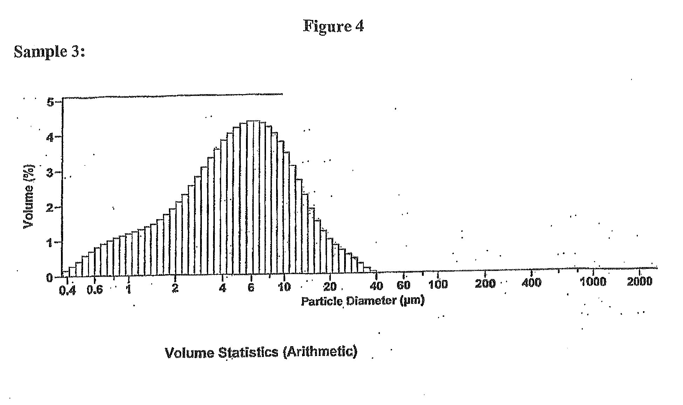

Figure 4 depicts and the data below tabulate for Sample 3 the arithmetic statistics describing surface area, particle size, and cumulative volume characteristics for a third lot of drug substance (feed material). The particle size distribution for this lot is depicted in Figure 4.

**Sample 3:**

Optical model:    Fraunhofer.rfz
LS 200                Small Volume Module

Calculations from 0.375 μm to 2000 μm

Volume:                100%
Mean:                   8.399 μm
Median:                 6.679 μm
Specific Surf. Area:    16802 cm$^2$/mL

| %< | 10 | 25 | 50 | 75 | 100 |
|---|---|---|---|---|---|
| μm | 1.498 | 3.385 | 6.679 | 11.50 | 63.41 |

Table 7

| Particle Diameter (Lower) μm | Cum. < Volume % | Diff. Volume % | Particle Diameter (Lower) μm | Cum. < Volume | Diff. Volume |
|---|---|---|---|---|---|
| 0.375 | 0 | 2.13 | 33.01 | 99.2 | 0.79 |
| 0.656 | 2.13 | 4.92 | 57.77 | 99.997 | 0.0029 |
| 1.149 | 7.05 | 6.97 | 101.1 | 100 | 0 |
| 2.011 | 14.0 | 12.1 | 176.9 | 100 | 0 |
| 3.519 | 26.1 | 20.4 | 309.6 | 100 | 0 |
| 6.158 | 46.4 | 25.7 | 541.9 | 100 | 0 |
| 10.78 | 72.2 | 19.8 | 948.3 | 100 | 0 |
| 18.86 | 92.0 | 7.20 | 1660 | 100 | 0 |

## EXAMPLE 5

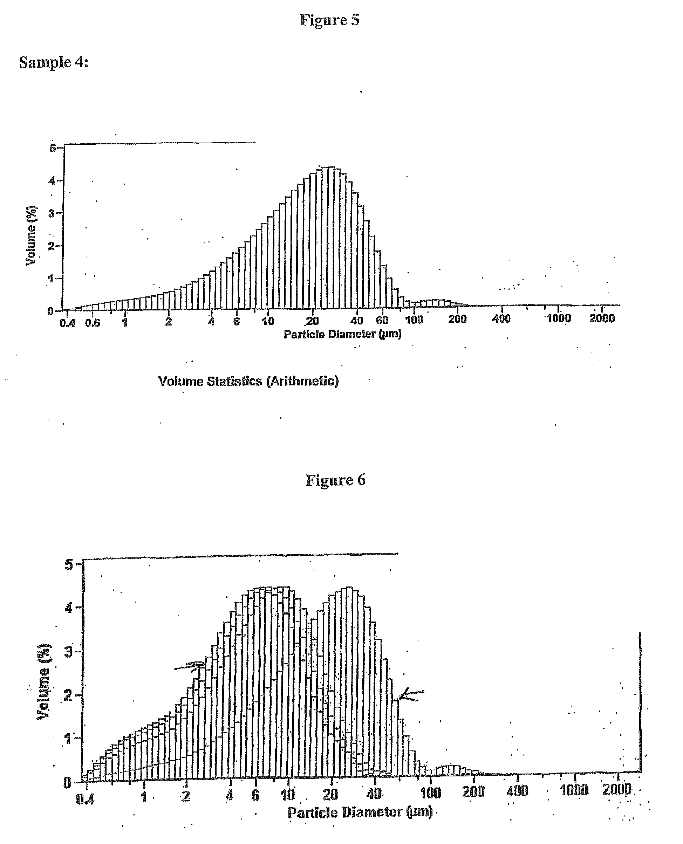

Figure 5 depicts and the data below tabulate for Sample 4 the arithmetic statistics describing surface area, particle size, and cumulative volume characteristics for a fourth lot of drug substance (feed material). The particle size distribution for this lot is depicted in Figure 5.

**Sample 4:**

| | |
|---|---|
| Optical model: | Fraunhofer.rfz |
| LS 200 | Small Volume Module |

Calculations from 0.375 $\mu$m to 2000 $\mu$m

| | |
|---|---|
| Volume: | 100% |
| Mean: | 22.93 $\mu$m |
| Median: | 17.82 $\mu$m |
| Specific Surf. Area: | 7553 cm$^2$/mL |

| < | 10 | 25 | 50 | 75 | 100 |
|---|---|---|---|---|---|
| $\mu$m | 3.851 | 8.660 | 17.82 | 30.86 | 309.6 |

Table 8

| Particle Diameter (Lower) $\mu$m | Cum. < Volume % | Diff. Volume % | Particle Diameter (Lower) $\mu$m | Cum. < Volume | Diff. Volume |
|---|---|---|---|---|---|
| 0.375 | 0 | 0.61 | 33.01 | 78.0 | 17.2 |
| 0.656 | 0.61 | 1.55 | 57.77 | 95.3 | 3.43 |
| 1.149 | 2.17 | 2.46 | 101.1 | 98.7 | 1.08 |
| 2.011 | 4.62 | 4.37 | 176.9 | 99.8 | 0.24 |
| 3.519 | 8.99 | 8.23 | 309.6 | 100 | 0 |
| 6.158 | 17.2 | 14.1 | 541.9 | 100 | 0 |
| 10.78 | 31.3 | 21.2 | 948.3 | 100 | 0 |
| 18.86 | 52.5 | 25.5 | 1660 | 100 | 0 |

**Figure 6** depicts a comparison of particle size distributions from four lots of drug substance (feed material). The data indicates a distinct physical difference in these four lots of drug substance with a range of mean particle size from 6.503 $\mu$m to 22.93 $\mu$m.
Table 9 depicts the physical characteristics of three lots of drug substance (feed powder samples) and the corresponding drug product (encapsulated granulation samples) using these same lots of drug substance.

Table 9:

| FEED POWDER SAMPLES | | | | | | | | |
|---|---|---|---|---|---|---|---|---|
| PRODUCT | MOISTURE - L.O.D. | LBD g/cc | TBD g/cc | Angle of Repose | Compression 20K | API Particle Size (Microns) 100%< | Median | Mean |
| Sample_ (Omni Chem) | 0.15% | 0.46 | 0.6 | 45 | 1.28g/cc | 373 | 19.1 | 28.1 |
| Sample_ (Noveon 35) | 0.10% | 0.46 | 0.63 | 50 | 1.47g/cc | 83.9 | 8.0 | 10.8 |
| Sample_ (Noveon 53) | 0.10% | 0.43 | 0.57 | 60 | 1.32g/cc | 69.6 | 6.9 | 8.7 |
| | | | | | | | | |

| ENCAPSULATED GRANULATION SAMPLES | | | | | |
|---|---|---|---|---|---|
| PRODUCT | PARTICLE SIZES (MICRONS) | | LBD glee | Hardness (Retention) | Specific Surface Area cm$^2$/ml |
| | 100 % < Median Mean | | | | |
| Sample_ (Omni Chem) | 2000 695.3 | 650.6 | 0.67 | 89.2 + 60 Mesh | 3535 |
| Sample_ (Noveon 35) | 1660 428.0 | 427.9 | 0.69 | 85.8 + 60 Mesh | 5700 |
| Sample_ (Noveon 53) | 1512 194.3 | 315.9 | 0.68 | 86.5 + 60 Mesh | 8399 |

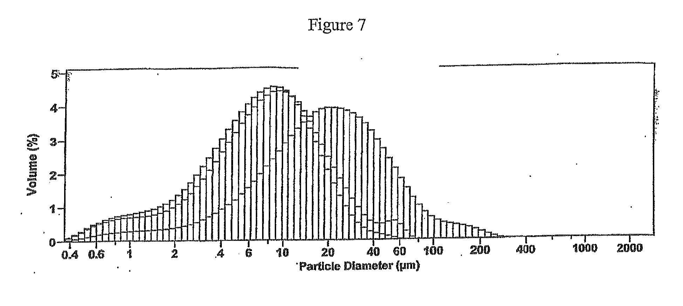

Figure 7 depicts a comparison of particle size distributions from three lots of drug substance (feed material). The data indicates a distinct physical difference in these three lots of drug substance with a range of mean particle size from 8.721 μm to 28.10 μm.

The data indicates a distinct physical difference in these four lots of drug substance with a range of mean particle size from 6.503 μm to 22.93 μm.

Table 10

| File name: | Balsalazide - Feed Lot #35.$01 |
|---|---|
| Group ID: | Balsalazide - Feed Lot #35 |
| Sample ID: | Salix - EL05 - 020105 |
| Run number: | 1 |
| Optical model: | Fraunhofer.rfz |
| LS 200 | Small Volume Module |
| | |
| File name: | Balsalazide - Feed Lot #41.$02 |
| Group ID: | Balsalazide - Feed Lot #41 |

(continued)

| Sample ID: | Salix - EL05 - 020105 |
|---|---|
| Run number: | 2 |
| Optical model: | Fraunhofer.rfz |
| LS 200 | Small Volume Module |
| | |
| File name: | Balsalazide - Feed Lot #53.$02 |
| Group ID: | Balsalazide - Feed Lot #53 |
| Sample ID: | Salix Pharm EL05 - 020105 |
| Run number: | 2 |
| Optical model: | Fraunhofer.rfz |
| LS 200 | Small Volume Module |

Table 11 A - D tabulates the arithmetic statistics describing surface area, particle size, and cumulative volume characteristics for three lots of drug substance (feed material). The particle size distributions for these lots are depicted in Figure 7. This data confirms the inverse correlation of smaller particle size associated with larger surface area. Of these three lots of drug substance, 100% of the particles are less than 83.89 $\mu$m, 373.1 $\mu$m, and 69.61 $\mu$m, respectively.

Table 11A

| Volume Statistics (Arithmetic) | | Balsalazide Feed Powder Sample A (#35.$01) | | | |
|---|---|---|---|---|---|
| | | | | | |
| Calculations from 0.375 $\mu$m to 2000 $\mu$m | | | | | |
| Volume: | 100% | | | | |
| Mean: | 10.85 $\mu$m | | | | |
| Median: | 8.031 $\mu$m | | | | |
| Specific Surf. Area: | 14230 cm$^2$/mL | | | | |
| % c | 10 | 25 | 50 | 75 | 100 |
| $\mu$m | 1.899 | 4.253 | 8.031 | 13.88 | 83.89 |

Table 11 B

| Volume Statistics (Arithmetic) | Balsalazide - Feed Powder Sample B (#41.$02) | | | | |
|---|---|---|---|---|---|
| Calculations from 0.375 $\mu$m to 2000 $\mu$m | | | | | |
| Volume: | 100% | | | | |
| Mean: | 28.10 $\mu$m | | | | |
| Median: | 19.10 $\mu$m | | | | |
| Specific Surf. Area: | 6894 cm$^2$/mL | | | | |
| %< | 10 | 25 | 50 | 75 | 100 |
| $\mu$m | 4.638 | 9.736 | 19.10 | 35.30 | 373.1 |

Table 11 C

| Volume Statistics (Arithmetic) | | Balsalazide - Feed Powder Sample B (#41.$02) | |
|---|---|---|---|
| Calculations from 0.375 $\mu$m to 2000 $\mu$m | | | |
| Volume: | 100% | | |
| Mean: | 8.721 $\mu$m | | |
| Median: | 6.993 $\mu$m | | |
| Specific Surf. Area: | 15822 cm$^2$/mL | | |
| % < 10 | 25 | 50 | 75 | 100 |
| $\mu$m 1.664 | 3.686 | 6.993 | 11.80 | 69.61 |

Table 11 D

| | Balsalazide | | | Balsalazide | | | Balsalazide | |
|---|---|---|---|---|---|---|---|---|
| Particle | - Feed Powder | Diff. | | - Feed Powder | Diff. | | - Feed Powder | Diff. |
| Diameter | Sample A (#35.$01) | Volume | | Sample B (#41.$02) | Volume | | Sample C (#53.$02) | Volume |
| (Lower) | Cum. < | % | | Cum. < | % | | Cum. < | % |
| $\mu$m | Volume | | | Volume | | | Volume | |
| | % | | | % | | | % | |
| 0.375 | 0 | 1.82 | | 0 | 0.64 | | 0 | 1.96 |
| 0.656 | 1.82 | 3.88 | | 0.64 | 1.50 | | 1.96 | 4.37 |
| 1.149 | 5.70 | 4.90 | | 2.15 | 1.94 | | 6.33 | 6.03 |
| 2.011 | 10.6 | 9.30 | | 4.08 | 3.16 | | 12.4 | 11.3 |
| 3.519 | 19.9 | 18.4 | | 7.24 | 6.98 | | 23.7 | 20.6 |
| 6.158 | 38.3 | 25.7 | | 14.2 | 13.9 | | 44.2 | 26.7 |
| 10.78 | 63.9 | 21.8 | | 28.1 | 21.3 | | 70.9 | 20.7 |
| 18.86 | 85.7 | 10.2 | | 49.5 | 23.1 | | 91:6 | 7.30 |
| 33.01 | 95.9 | 3.32 | | 72.5 | 17.0 | | 98.9 | 1.07 |
| 57.77 | 99.3 | 0.75 | | 89.5 | 7.10 | | 99.99 | 0.013 |
| 101.1 | 100 | 0 | | 96.6 | 2.65 | | 100 | 0 |
| 176.9 | 100 | 0 | | 99.3 | 0.73 | | 100 | 0 |
| 309.6 | 100 | 0 | | 99.999 | 0.0011 | | 100 | 0 |
| 541.9 | 100 | 0 | | 100 | 0 | | 100 | 0 |
| 948.3 | 100 | 0 | | 100 | 0 | | 100 | 0 |
| 1660 | 100 | 0 | | 100 | 0 | | 100 | 0 |

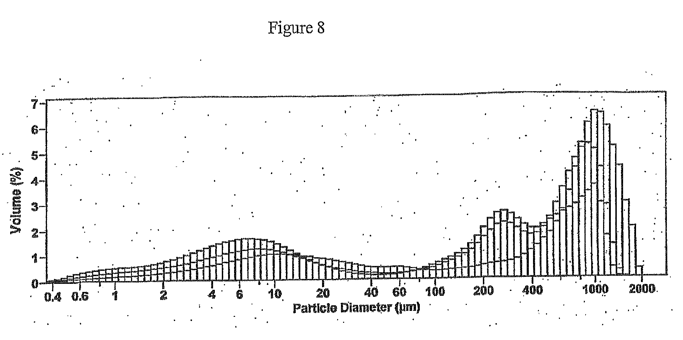

Table 12 tabulates the arithmetic statistics describing surface area, particle size, and cumulative volume characteristics for three lots of drug product (encapsulated granulation samples) that were manufactured with the three lots of drug substance (feed material) as depicted in Figure 7. The data indicates that the granules in these three lots of drug product have a range of mean particle size of 315.9 $\mu$m to 650.6 $\mu$m. Of these three lots of drug product, 100% of the granules are less than 1660 $\mu$m, 2000 $\mu$m, and 1512 $\mu$m, respectively. The particle size distributions for these three lots are depicted in Figure 8. Despite the distinct physical differences in the drug substance, the manufacturing process for the drug product is such that these differences have been minimized and the dissolution profiles of drug product are

**EP 2 529 729 A1**

comparable in their respective rates of dissolution.

Table 12

| Encapsulated Sample 1 | | | | |
|---|---|---|---|---|
| Volume Statistics (Arithmetic) | | Balsalazide - Product Lot #35.$04 | | |
| Calculations from 0.375 $\mu$m to 2000 $\mu$m | | | | |
| Volume: | | 100% | | |
| Mean: | | 427.9 $\mu$m | | |
| Median: | | 318.7 $\mu$m | | |
| Specific Surf. Area: | | 5700 cm$^2$/mL | | |
| % < | 10 | 25 | 50 | 75 | 100 |
| $\mu$m | 3.890 | 13.86 | 318.7 | 770.6 | 1660 |
| Encapsulated Sample 2 | | | | |
| Volume Statistics (Arithmetic) | | Balsalazide - Product Lot #41.$04 | | |
| Calculations from 0.375 $\mu$m to 2000 $\mu$m | | | | |
| Volume:<br>Mean: | | 100%<br>650.6 $\mu$m | | |
| Median: | | 695.3 $\mu$m | | |
| Specific Surf. Area: | | 3535 cm$^2$/ML | | |
| % < | 10 | 25 | 50 | 75 | 100 |
| $\mu$m | 6.747 | 33.83 | 695.3 | 1072 | 2000 |
| | | | | |
| Encapsulated Sample 3 | | | | |
| Volume Statistics (Arithmetic) | | Balsalazide - Product Lot #53.$05 | | |
| Calculations from 0.375 $\mu$m to 2000 $\mu$m | | | | |
| Volume: | 100% | | | |
| Mean: | 31 5.9 $\mu$m | | | |
| Median: | 194.3 $\mu$m | | | |
| Specific Surf. Area: | 8399 cm$^2$/mL | | | |
| % < | 10 | 25 | 50 | 75 | 100 |
| $\mu$m | 2.574 | 7.214 | 194.3 | 554.2 | 1512 |

The disclosures of each and every patent, patent application and publication cited herein are hereby incorporated herein by reference in their entirety.

While the invention has been disclosed with reference to specific embodiments, it is apparent that other embodiments and variations of the invention may be devised by others skilled in the art without departing from the true spirit and scope of the invention. The appended claims are intended to be construed to include all such embodiments and equivalent variations.

**Claims**

1.  A method of manufacturing a capsule, comprising:

    granulating the balsalazide disodium and one or more excipients to form granules;

sizing the granules;
blending the granules for about 20 minutes to form a powder blend; and
encapsulating the powder blend.

2. The method of claim 1, wherein the one or more excipients comprise one or more of colloidal silicon dioxide or magnesium stearate.

3. The method of claim 1, wherein the excipients comprise from between about 2 to about 3% of the granules by weight.

4. The method of claim 1, wherein the sizing is with a 12 mesh screen.

5. The method of claim 1, wherein the blending is with a double cone blender.

6. The method of claim 1, further comprising polishing the encapsulated powder blend.

7. The method of claim 1, wherein from between about 700 mg and about 1200 mg of powder blend is encapsulated.

8. A pharmaceutical preparation of balsalazide made by the process, comprising:

    granulating a balsalazide disodium feed powder and one or more excipients to form granules;
    blending the granules for about 20 minutes to form a powder blend; and encapsulating the powder blend,

    wherein the two daily doses of about 6 grams per day of balsalazide or a pharmaceutically acceptable prodrug, salt, solvate, or clathrate thereof are administered.

9. The pharmaceutical preparation of claim 8, wherein the method further comprises sizing the granules before blending.

10. The pharmaceutical preparation of claim 8, wherein the feed powder has an average particle diameter of from between about 8 to about 30 $\mu$m.

11. The pharmaceutical preparation of claim 8, wherein the powder blend has an average particle diameter of from about 300 to about 675 $\mu$m.

12. The pharmaceutical preparation of claim 8, wherein the powder blend has a specific surface area of from between about 3500 to about 8500 cm$^2$/ml.

13. The pharmaceutical preparation of claim 8, wherein the powder blend has a hardness (retention) of from between about 85 to about 90 (60 Mesh).

14. The pharmaceutical preparation of claim 8, wherein the powder blend has a Loose Bulk Density (LBD) of from between about 0.67 to about 0.69 g/cc.

15. The pharmaceutical preparation of claim 8, wherein the balsalazide disodium feed powder has a compression 20 K of from between about 1.28 to about 1.47 g/cc.

16. The pharmaceutical preparation of claim 8, wherein the balsalazide disodium feed powder has a Tapped Bulk Density (TBD) of from between about 0.57 to about 0.63 g/cc.

17. The pharmaceutical preparation of claim 8, wherein the balsalazide disodium feed powder has a moisture of from between about 0.10% and about 0.15%.

18. A pharmaceutical preparation of balsalazide made by the process, comprising:

    granulating the balsalazide disodium and one or more excipients to form granules;
    blending the granules for about 20 minutes to form a powder blend; and encapsulating the powder blend,

    wherein the powder blend has an average particle diameter of from between about 300 to about 675 $\mu$m.

19. The pharmaceutical preparation of claim 18, wherein the method further comprises sizing the granules before blending.

20. The pharmaceutical preparation of claim 18, wherein the method further comprises sizing the granules before blending.

21. The pharmaceutical preparation of claim 18, wherein the feed powder has an average particle diameter of from between about 8 to about 30 $\mu$m.

22. The pharmaceutical preparation of claim 18, wherein the powder blend has a specific surface area of from between about 3500 to about 8500 cm$^2$/ml.

23. The pharmaceutical preparation of claim 18, wherein the powder blend has a hardness (retention) of from between about 85 to about 90 (60 Mesh).

24. The pharmaceutical preparation of claim 18, wherein the powder blend has a Loose Bulk Density (LBD) of from between about 0.67 to about 0.69 g/cc.

25. The pharmaceutical preparation of claim 18, wherein the balsalazide disodium feed powder has a compression 20 K of from between about 1.28 to about 1.47 g/cc.

26. The pharmaceutical preparation of claim 18, wherein the balsalazide disodium feed powder has a Tapped Bulk Density (TBD) of from between about 0.57 to about 0.63 g/cc.

27. The pharmaceutical preparation of claim 18, wherein the balsalazide disodium feed powder has a moisture of from between about 0.10% and about 0.15%.

## Figure 1

| In-Process and Release Controls | Manufacturing Process | Validation Controls |
|---|---|---|

*Raw materials meet release specifications* → *Dispensing balsalazide disodium and (2) excipients* ← *Distinct lot of active for each validation batch*

*"SnapTest" for appropriate compact hardness* → *Granulating balsalazide disodium via roller compaction*

*Sizing of granules and (2) excipients through 12 mesh screen*

*Post-final blend sample meets tapped density specs.* → *Blending of granules and (2) excipients in Gemco-blender* ← *Blend uniformity testing via tapped density and assay*

*Encapsulating final blend into hard gelatin 00 capsules via PD-8*

*Finished product meets release specifications* → *Polishing of filled hard gelatin 00 capsules* ← *Beginning, middle, and end release testing*

Figure 2

Sample 1

Volume Statistics (Arithmetic)

Figure 3

Sample 2:

Volume Statistics (Arithmetic)

Figure 4

Sample 3:

Volume Statistics (Arithmetic)

Figure 5

Sample 4:

Volume Statistics (Arithmetic)

Figure 6

Figure 7

Figure 8

European
Patentamt

European
Patent Office

Office européen
des brevets

# EUROPEAN SEARCH REPORT

Application Number

EP 12 16 6566

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/074908 A1 (BORODY THOMAS J [AU]; SHORTIS NICOLAS PETER [AU]) 18 August 2005 (2005-08-18) * sentences 25-28, paragraph 8 * ----- | 1-27 | INV. A61K9/14 A61K9/16 A61K9/48 A61K31/609 |
| Y | US 6 562 871 B1 (CAPPOLA MICHAEL L [US]) 13 May 2003 (2003-05-13) * the whole document * ----- | 1-27 | |
| Y | WO 2005/021009 A2 (AGI THERAPEUTICS LTD [IE]; DEVANE JOHN [IE]; BUTLER JACKIE [IE]) 10 March 2005 (2005-03-10) * paragraphs [0044], [0186] - [0198], [0338] - [0349] * ----- | 1-27 | |

TECHNICAL FIELDS
SEARCHED        (IPC)

A61K

The present search report has been drawn up for all claims

| Place of search | Date of completion of the search | Examiner |
|---|---|---|
| Munich | 29 October 2012 | Ansaldo, M |

EPO FORM 1503 03.82 (P04C01)

**ANNEX TO THE EUROPEAN SEARCH REPORT
ON EUROPEAN PATENT APPLICATION NO.**

EP 12 16 6566

This annex lists the patent family members relating to the patent documents cited in the above-mentioned European search report.
The members are as contained in the European Patent Office EDP file on
The European Patent Office is in no way liable for these particulars which are merely given for the purpose of information.

29-10-2012

| Patent document cited in search report | | Publication date | Patent family member(s) | | Publication date |
|---|---|---|---|---|---|
| WO 2005074908 | A1 | 18-08-2005 | AT | 554752 T | 15-05-2012 |
| | | | CA | 2555304 A1 | 18-08-2005 |
| | | | EP | 1720536 A1 | 15-11-2006 |
| | | | EP | 2361620 A1 | 31-08-2011 |
| | | | JP | 2007520495 A | 26-07-2007 |
| | | | JP | 2012102112 A | 31-05-2012 |
| | | | NZ | 549661 A | 30-07-2010 |
| | | | US | 2007213304 A1 | 13-09-2007 |
| | | | WO | 2005074908 A1 | 18-08-2005 |
| | | | ZA | 200607435 A | 27-05-2009 |
| US 6562871 | B1 | 13-05-2003 | NONE | | |
| WO 2005021009 | A2 | 10-03-2005 | AT | 489956 T | 15-12-2010 |
| | | | AU | 2004267953 A1 | 10-03-2005 |
| | | | CA | 2535923 A1 | 10-03-2005 |
| | | | EP | 1663253 A2 | 07-06-2006 |
| | | | ES | 2353761 T3 | 04-03-2011 |
| | | | JP | 2007504212 A | 01-03-2007 |
| | | | MX | PA06002048 A | 25-05-2006 |
| | | | US | 2005090473 A1 | 28-04-2005 |
| | | | US | 2010278917 A1 | 04-11-2010 |
| | | | WO | 2005021009 A2 | 10-03-2005 |
| | | | ZA | 200601356 A | 25-07-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9518622 A
- US 6197341 B
- US 6326364 B

**Non-patent literature cited in the description**

- Remington's Pharmaceutical Sciences. Mack Publishing Company, 1990, 1658-1659
- Analgesic-Antipyretic and Antiinflammatory Agents and Drugs Employed in the Treatment of Gout. **PAUL A. INSEL.** Goodman & Gilman's The Pharmacological Basis of Therapeutics. 1996, 617-57
- Analgesic, Antipyretic and Anti-Inflammatory Drugs. **GLEN R. HANSON.** Remington: The Science and Practice of Pharmacy. 1995, vol. II, 1196-1221
- **P. KERLIN ; L. WONG.** Breath hydrogen testing in bacterial overgrowth of the small intestine. *Gastroenterol.,* 1988, vol. 95 (4), 982-88
- **A. STROCCHI et al.** Detection of malabsorption of low doses of carbohydrate: accuracy of various breath H2 criteria. *Gastroenterol.,* 1993, vol. 105 (5), 1404-1410
- **G. R. SWART ; J. W. VAN DEN BERG.** C breath test in gastrointestinal practice. *Scand. J. Gastroenterol.,* 1998, vol. 225, 13-18
- **C. E. KING ; P. P. TOSKES.** Breath tests in the diagnosis of small intestinal bacterial overgrowth. *Crit. Rev. Lab. Sci.,* 1984, vol. 21 (3), 269-81
- **C. S. CHANG et al.** Increased accuracy of the carbon-14D-xylose breath test in detecting small-intestinal bacterial overgrowth by correction with the gastric emptying rate. *Eur. J. Nucl. Med.,* 1995, vol. 22 (10), 1118-22
- **A. SCHNEIDER et al.** Value of the C-D-xylose breath test in patients with intestinal bacterial overgrowth. *Digestion,* 1985, vol. 32 (2), 86-91